# EUROPÄISCHE PATENTANMELDUNG

(11) **EP 3 173 036 A1**
(43) Veröffentlichungstag der Anmeldung: **31.05.2017**
(21) Anmeldenummer: 15196654.6
(22) Anmeldetag: 27.11.2015
(51) Int. Cl.: A61B 17/16, A61B 17/17, A61B 17/32, A61B 17/00

(54) **VORRICHTUNG ZUM ENTFERNEN VON KNOCHEN- BZW. BINDEGEWEBE, INSBESONDERE ZUR DEKOMPRESSION DES WIRBELKANALS BEI SPINALSTENOSE**

(71) Anmelder: NES-Med GmbH, 6300 Zug (CH)
(72) Erfinder: Harren, Ernst-Diethelm, CH-6343 Rotkreuz (CH)
(74) Vertreter: Luchs, Willi

(57) **Zusammenfassung**

Die Erfindung betrifft eine Vorrichtung zum Entfernen von Knochen- und Bindegewebe, insbesondere zur Dekompression des Wirbelkanals bei Spinalstenose, mit einem entlang eines gekrümmten Leitpfads hin- und herbewegbaren Abtragungselement (1), das mit einem ihn im Bereich des Zielgewebes (9) positionierenden Führungsinstrument (2) zusammenwirkt. Letzteres besteht insbesondere aus einem biegsamen Röhrchen (8), das von Hand in den zu behandelnden Bereich einführbar und in situ dem Verlauf des gekrümmten Pfades um das Zielgewebe (9) herum anpassbar ist, wobei das Röhrchen (8) nach Positionieren des darin geführten Abtragungselementes (1) wieder von Hand aus dem zu behandelnden Bereich ausziehbar ist, während das Abtragungselement (1) die dort eingenommene Lage beibehält. Die Anpassung des Röhrchens an den Verlauf des jeweils gewählten Leitpfads kann in situ von Hand durch zwei- oder dreidimensionales Umbiegen des Röhrchens (8) durchgeführt werden.

## Beschreibung

Die Erfindung betrifft eine Vorrichtung zum Entfernen von Knochen- und/oder Bindegewebe, insbesondere zur Dekompression des Wirbelkanals bei Spinalstenose, mit einem entlang eines gekrümmten Leitpfades hin- und herbewegbaren Abtragungselement, das mit einem ihn im Bereich des Zielgewebes positionierenden Führungsinstrument zusammenwirkt.

Vorrichtungen dieser eingangs erwähnten Gattung dienen zur Entlastung des Wirbelkanals, wenn dieser bei Spinalstenose etwa infolge von Verschleisserscheinungen oder durch die Verdickung der Wirbelgelenke infolge von Arthrose zu sehr verengt ist. Die operative Entlastung des Wirbelkanals wird durch Entfernen des überwachsenen Bindegewebes und/oder Knochenmaterials mittels einem spanabhebenden Abtragungselement bewirkt, welches im Lendenwirbelbereich entlang des Zielgewebes hin- und herbewegt wird.

Eine Vorrichtung dieser Art ist in der Druckschrift US PS 7,887,538 geoffenbart. Bei ihr erfolgt die Einführung des Abtragungselementes über ein starres Führungsrohr, das geradlinig bis in die Nähe des zu behandelnden Bereiches sich erstreckt und in dieser Lage verbleibt, bis die Operation beendet ist. Das starre Führungsrohr bewirkt eine ungenaue Positionierung des Abtragungselementes im zu behandelnden Bereich, der ausserhalb des Führungsrohres nur durch einen engen Leitpfad mit individuell unterschiedlichem Verlauf erreicht wird.

Der Erfindung liegt demgegenüber die Aufgabe zugrunde, diesen Nachteil zu vermeiden und eine Vorrichtung der eingangs genannten Gattung zu schaffen, die eine einfache und genaue Positionierung des Abtragungselementes in situ ermöglicht.

Diese Aufgabe wird erfindungsgemäss dadurch gelöst, dass das Führungsinstrument der Vorrichtung aus einem biegsamen Material besteht, das in den zu behandelnden Bereich einführbar und in situ dem Verlauf des gekrümmten Leitpfades um das Zielgewebe herum anpassbar ist, wobei das Röhrchen nach Positionieren des darin geführten Abtragungselementes wieder aus dem zu behandelnden Bereich ausziehbar ist, während das Abtragungselement darin verbleibt.

Durch die Biegsamkeit des vorzugsweise als Röhrchen gebildeten erfindungsgemässen Führungsinstruments wird ermöglicht, dass dieses in situ von Hand entsprechend der Geometrie des gekrümmten Leitpfads so umgebogen werden kann, das es gefühlvoll in den Bereich des Zielgewebes einschiebbar ist.

Das Abtragungselement kann dabei zusammen mit ihm oder nachträglich durch das Führungsinstrument geschoben werden. Danach wird das Führungsinstrument wieder aus dem zu behandelnden Bereich herausgezogen, während das Abtragungselement gegen das Zielgewebe angelegt bleibt und das überschüssige Bindegewebe-, Span- und/oder Knochenmaterial mittels einer Hin- und Herbewegung abtragen kann.

Es ist zur Erleichterung der Handhabung zweckmässig, wenn das als Führungsinstrument dienende Röhrchen mit einem längeren Schaftteil zum Halten des Führungsinstruments von Hand sowie einem vorzugsweise sichelförmigen, kürzeren Führungsteil zum Einführen des Röhrchens in den zu behandelnden Bereich versehen ist.

Das sichelförmige kürzere Führungsteil kann durch Zug- und Druckbewegung am proximalen Ende der Vorrichtung aus dem Leitpfad heraus navigiert werden.

Das Röhrchen ist vorzugsweise aus einem nicht oxidierenden Metall, wie rostfreiem Stahl, einer für die Medizintechnik geeigneten Metallegierung, wie NiTi, CoCr oder ähnlichem, oder aus einem Kunststoff, vorzugsweise einem Thermoplast, mit einem Aussendurchmesser von vorzugsweise 1 bis 4 mm hergestellt. Es ist dabei so biegsam hergestellt, dass es von Hand mit einer von Fall zu Fall unterschiedlichen Krümmung biegbar ist, aber dennoch mit einer ausreichenden Stabilität versehen ist, damit es sich beim Einführen des Röhrchens in den zu behandelnden Bereich nicht verbiegt.

Das erfindungsgemässe Röhrchen weist einen Querschnitt auf, dessen Form und Abmessungen dem Querschnitt des Abtragungselementes angepasst sind. Um die Einführung des Röhrchens in den zu behandelnden Bereich zu erleichtern, sind möglichst enge und runde Querschnitte bevorzugt. In Fällen, wo unterschiedlich dimensionierte Abtragungselemente zum Einsatz kommen, kann die Bereitstellung eines Satzes von Führungsinstrumenten mit entsprechend ausgestalteten Röhrchen vorteilhaft sein.

Die Erfindung sieht ferner vor, dass sich das Abtragungselement zusammen aus einem vorzugsweise zweiteiligen Einzugsdraht und einem mit ihm verbindbaren spanabhebenden Element in Gestalt eines flexiblen Längsbandes aus einer Metallfolie mit einer Schleifmittelbeschichtung zusammensetzt. Letztere ist so beschaffen, dass das Spanabhebungsmaterial härter ist als das abzutragende Gewebe.

Der zweiteilige Einzugsdraht besteht aus zwei als Einzugshilfen wirkenden Einzeldrähten, die an je einem Ende der spanabhebenden Metallbandes verbindbar sind und dieses in an sich bekannter Weise entlang des Zielgewebes hin und herziehen können. Die Verbindung zwischen der bandförmigen Metallfolie und den Einzugsdrähten kann auch nach Einführen des Abtragungselementes in den zu behandelnden Bereich hergestellt werden.

Um eine schnelle und sichere Verbindung zu ermöglichen, sieht die Erfindung vor, dass die Einzugsdrähte mit Kupplungselementen in Gestalt von an den Drahtenden befestigen Kugeln zur Aufnahme des spanabhebendes Elements versehen sind, welche sich an dessen Enden einklinken können und so ausgebildet sind, dass sie die Aufnahme unterschiedlich beschaffener oder dimensionierter Elemente ermöglichen.

Die Erfindung ist nachfolgend anhand von Ausführungsbeispielen unter Bezugnahme auf die Zeichnung näher erläutert. Es zeigen:
- Fig. 1: einen schematischen Querschnitt einer Wirbelsäule mit einer erfindungsgemässen Vorrichtung zur Dekompression des Wirbelkanals bei Spinalstenose;
- Fig. 2: eine Ansicht eines biegsamen Führungsinstrumentes der Vorrichtung aus Fig. 1; und
- Fig. 3: ein Abtragungselement der Vorrichtung nach Fig. 1, perspektivisch dargestellt.

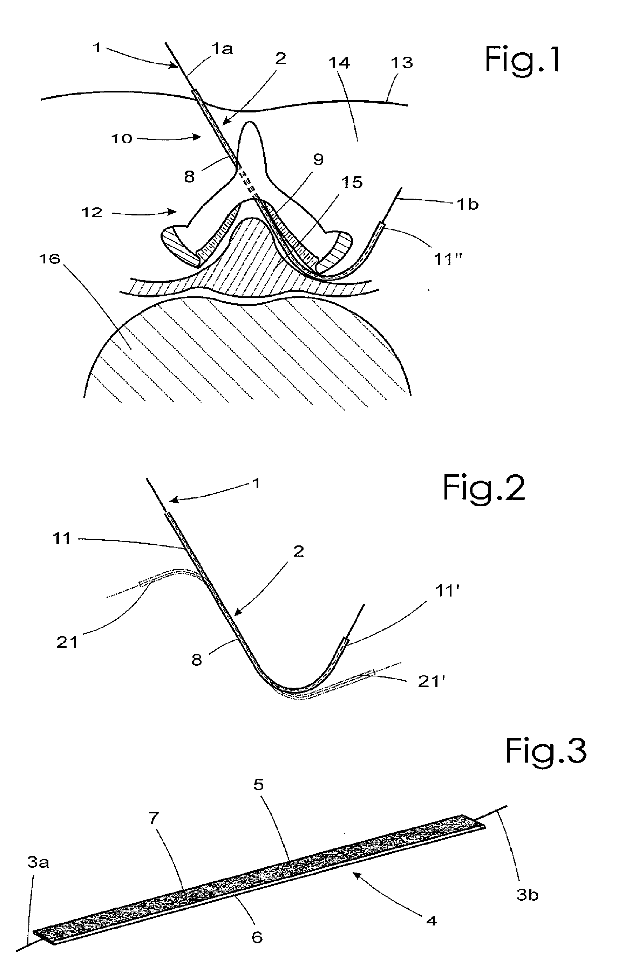

Fig. 1 zeigt schematisch einen Querschnitt einer menschlichen Wirbelsäule 12 mit einem Wirbelkörper 16, welche in Muskelgewebe 14 und der Aussenhaut 13 eingebettet sind. Zudem ist zwischen der Wirbelsäule 12 und dem Wirbelkörper 16 der Wirbelkanal 15 und das Zielgewebe 9 angedeutet.

Die erfindungsgemässe Vorrichtung 10 ist entlang eines gekrümmten Leitpfades zwischen dem Zielgewebe 9 und dem Wirbelkanal 15 eingeführt. Sie eignet sich insbesondere für die Dekompression des Wirbeikanals 15 bei Spinalstenose, da es wegen des die Wirbelsäule 12 umschliessenden Muskelgewebes besonders schwierig ist, sicher in den Bereich des zu behandelnden Zielgewebes zu gelangen.

Die Vorrichtung 10 eignet sich aber generell zum Abtragen von Gewebe- und/oder Knochenmaterial überall dort, wo das Zielgewebe 9 nur über einen zwei- oder dreidimensional gekrümmten Leitpfad erreicht wird.

Die Vorrichtung 10 weist ein Führungsinstrument 2 auf, das in situ von Hand in den zu behandelnden Bereich einführbar und dem Verlauf des gekrümmten Leitpfades um das Zielgewebe 9 herum anpassbar ist. Dieses Führungsinstrument 2 wird nach Positionieren im zu behandelnden Bereich wieder herausgezogen, während das darin geführte Einzugsdraht 1 bzw. ein Abtragungselement die dort eingenommene Lage beibehält.

Bei Verwendung eines in das Führungsinstrument 2 eingeführten Einzugsdrahtes 1 kann dieser nach dem Herausziehen des Führungsinstrumentes durch Festhalten seines gegenüberliegenden Endes 1 b zu der Ausziehrichtung im Leitpfad belassen werden. Danach kann dieser Einzugsdraht 1 am einen Ende 1a mit einem Abtragungselement 4 für das Abtragen verbunden und dann sein anderes Ende 1b gezogen werden, bis das Abtragungselement 4 in dem Leitpfad eingeführt ist und mit diesem die Gewebe- bzw. Knochenentfernung wunschgemäss ausgeführt werden kann.

Das als Röhrchen 8 ausgebildete Führungsinstrument 2 wird, wie aus Fig. 1 ersichtlich, quer zur Wirbelsäule 12 durch die Haut 13 und das Muskelgewebe 14 entlang eines gekrümmten Leitpfades zwischen dem Zielgewebe 9 und dem Wirbelkanal 15 eingeführt. Da der Leitpfad in der Regel dreidimensional gekrümmt ist, hat das Röhrchen 8 zweckmässigerweise einen kreisförmigen Querschnitt, das die Biegsamkeit des Röhrchens in allen Richtungen erleichtert. Je nach Ausgestaltung des spanabhebenden Elements 4 können auch anders ausgebildete Rohrquerschnitte in Frage kommen. Um die Einführung des Röhrchens zu erleichtern, ist es vorteilhaft, wenn dieses mit einer atraumatischen Spitze 11" und dabei mit einem möglichst engen Querschnitt versehen ist, vorteilhaft zwischen 1 und 4 Millimetern.

Das erfindungsgemässe Röhrchen als Führungsinstrument kann mit oder ohne reibungsreduzierender äusserer Oberfläche ausgestattet sein. Eine Reibungsreduktion kann mittels geometrischen Formen oder durch das Aufbringen von entsprechenden Beschichtungen (hydrophil oder hydrophob) erfolgen.

Die erfindungsgemässe Vorrichtung zeichnet sich dadurch aus, dass sie einen einfachen Zugang zum Wurzelkanal gewährleistet und die Herstellung eines durch Zugkraft auf das spanabhebende Element 4 leicht individuell einstellbaren Drucks ermöglicht und somit punktuelle und flächige Korrekturen möglich macht, wobei nicht nur einer Verletzung der Nervenwurzeln sondern auch eine ungewollte übermässige Abtragung von Knochenmaterial und Bindegewebe verhindert wird.

Gemäss Fig. 2 ist das als Röhrchen 8 ausgebildete Führungsinstrument 2 mit einem längeren Schaftteil 11 zum Halten des Instruments von Hand versehen und weist auf der andern Seite einen kürzeren Führungsteil 11' auf, der annährend sichelförmig ausgebildet ist und dadurch das Einführen des Röhrchens 8 in den zu behandelnden Bereich besser ermöglicht wird.

Es ist zudem verdeutlicht, dass das Führungsinstrument 2 durch seine biegsame Fertigung unterschiedlich gekrümmt sein kann, je nachdem, wie der Leitpfadverlauf bei einer Wirbelsäule 12 ist. Es sind strichliniert abweichende Biegungen der beiden Rohrenden 21, 21' angedeutet. Dadurch ist es jederzeit möglich, die Krümmungen des Führungsinstruments 2 individuell in Anpassung an den Verlauf des jeweils günstigsten Leitpfads zu variieren bzw. bei einem Anstehen beim Einführen dieselben zu verändern.

Gemäss Fig. 3 ist der Vorrichtung 10 ein Abtragungselement 4 als eine Variante zugeordnet, das im eingesetzten Zustand entlang des gekrümmten Leitpfads bewegbar ist. Letzteres besteht aus zwei Einzugsdrähten 3a, 3b und einem mit ihnen verbundenen spanabhebenden Element in Gestalt eines flexiblen Längsbandes 5 aus einer Metallfolie 6 mit einer Schleifmittelbeschichtung 7, die so beschaffen ist, dass das spanabhebende Element durch die Hin- und Herbewegung des flexiblen Längsbandes 5 Bindegewebe- und/oder Knochenmaterial abtragen kann.

Die Verbindung zwischen dem Längsband 5 und den Einzugsdrähten 3a, 3b kann beispielsweise mittels an beiden Enden des Längsbandes 5 angeordneten nicht näher dargestellten Kugeln, die in die entsprechenden Enden des Längsbandes 5 einklinkbar und so ausgebildet sind, dass sie unterschiedlich beschaffene und/oder dimensionierte spanabhebende Elemente aufnehmen können.

Das Abtragungselement 4 kann zusammen mit dem Röhrchen 8 eingeführt werden oder nachträglich durch das Röhrchen 8 geschoben werden, wobei die Einzugsdrähte 3a, 3b beidseitig vorstehen und an entsprechend ausgebildete Halterungen anschliessbar sind, durch welche das Längsband 5 zum Abtragen des Zielgewebes 9 hin- und herbewegt wird. Davor wird aber das Röhrchen 8 wieder aus dem zu behandelnden Wirbelsäulenbereich herausgezogen, während das Abtragungselement 4 am Ende gehalten wird und in der jeweils eingenommenen Position sicher verbleibt.

Die Erfindung ist den obigen Ausführungsbeispielen ausreichend dargetan. Sie könnte aber noch durch weitere Varianten erläutert sein. So könnte das Führungsinstrument anstefle eines biegsamen runden Röhrchens aus einem bandförmigen Hohlkörper mit einem länglichen Innenquerschnitt oder ähnlichem gebildet sein.

Im Prinzip könnte vorzugsweise das vordere Ende des Führungsinstrumentes 2 mit einer medizinischen Kamera versehen sein, mittels welcher das Einführen des Röhrchens erleichtert bzw. der Zustand des Gewebes und der Wirbelsäule visualisiert werden könnten.

## Patentansprüche

1. Vorrichtung zum Entfernen von Knochen- und/oder Bindegewebe, insbesondere zur Dekompression des Wirbelkanals bei Spinalstenose, mit einem entlang eines gekrümmten Leitpfades hin-und herbewegbaren Abtragungselementes (1), das mit einem ihn im Bereich des Zielgewebes (9) positionierenden Führungsinstrument (2) zusammenwirkt, **dadurch gekennzeichnet, dass**
das Führungsinstrument (2) aus einem biegsamen Material besteht, das in den zu behandelnden Bereich einführbar und in situ dem Verlauf des gekrümmten Leitpfades um das Zielgewebe (9) herum anpassbar ist, wobei das Führungsinstrument (2) nach Positionieren des darin geführten Abtragungselementes (4) wieder aus dem zu behandelnden Bereich ausziehbar ist, während das Abtragungselement (4) darin verbleibt.

2. Vorrichtung nach Anspruch 1, **dadurch gekennzeichnet, dass** das Führungsinstrument (2) aus einem biegsamen Röhrchen (8) oder dergleichen Hohlkörper besteht.

3. Vorrichtung nach Anspruch 1 oder 2, **dadurch gekennzeichnet, dass**
dass Führungsinstrument (2) von Hand mit einer unterschiedlichen Krümmung biegbar ist, aber dennoch mit einer ausreichenden Stabilität versehen ist, damit es sich beim Einführen in den zu behandelnden Bereich nicht verbiegt.

4. Vorrichtung nach Anspruch 2 oder 3, **dadurch gekennzeichnet, dass**
das Röhrchen (8) mit einem längeren Schaftteil (11) zum Halten des Führungsinstruments (2) von Hand sowie einem vorzugsweise sichelförmigen, kürzeren Führungsteil (11') zum Einführen des Röhrchens in den zu behandelnden Bereich versehen ist.

5. Vorrichtung nach Anspruch 2, **dadurch gekennzeichnet, dass**
das Material des Röhrchens (8) aus rostfreiem Stahl, einer Metalllegierung, wie NiTi, CoCr, oder aus einem Kunststoff, vorzugsweise einem Thermoplast, mit einem Aussendurchmesser von vorzugsweise 1 bis 4 mm hergestellt ist.

6. Vorrichtung nach einem der vorhergehenden Ansprüche 2 bis 5, **dadurch gekennzeichnet, dass**
das Röhrchen (8) einen vorzugsweise runden Querschnitt aufweist, dessen innenseitige Form und Abmessungen dem Querschnitt des Abtragungselementes (4) bzw. eines Einzugsdrahtes (1) für dessen Aufnahme angepasst ist.

7. Vorrichtung nach einem der Ansprüche 1 bis 6, **dadurch gekennzeichnet, dass**
der Einzugsdraht (1) nach dem Herausziehen des Führungsinstrumentes (2) durch Festhalten im Leitpfad belastbar verbleibt, dass er am einen Ende mit einem Abtragungselement (4) verbindbar und sein anderes Ende herausziehbar ist, bis das Abtragungselement (4) in dem Leitpfad eingeführt ist und mit diesem die Gewebe- bzw. Knochenentfernung ausgeführt werden kann.

8. Vorrichtung nach einem der Ansprüche 1 bis 7, **dadurch gekennzeichnet, dass**
sich das Abtragungselement (4) aus einem vorzugsweise zweiteiligen Einzugsdraht (3a, 3b) und einem mit ihm verbindbaren spanabhebenden Element zusammensetzt.

9. Vorrichtung nach Anspruch 8, **dadurch gekennzeichnet, dass** das Abtragungselement (4) in Gestalt eines flexiblen Längsbandes (5) aus einer Metallfolie (6) mit einer Schleifmittelbeschichtung (7) gefertigt ist.

10. Vorrichtung nach Anspruch 8 oder 9, **dadurch gekennzeichnet, dass**
der Einzugsdraht (3a, 3b) zwei Einzeldrähte mit an ihren Enden befestigten Kugeln oder ähnlichem zur Aufnahme des spanabhebenden Elements (4) aufweist.

11. Vorrichtung nach einem der Ansprüche 1 bis 10, **dadurch gekennzeichnet, dass** die Spitze (11") des Führungsinstrumentes (2) atraumatisch ausgebildet ist.

12. Vorrichtung nach einem der Ansprüche 1 bis 11, **dadurch gekennzeichnet, dass** das vordere Ende des Führungsinstrumentes (2) mit einer Kamera versehen ist.
